# EUROPÄISCHE PATENTSCHRIFT

(11) **EP 2 675 817 B1**
(45) Veröffentlichungstag und Bekanntmachung des Hinweises auf die Patenterteilung: **16.09.2015**
(21) Anmeldenummer: 12705106.8
(22) Anmeldetag: 14.02.2012
(51) Int. Cl.: C07F 7/00, D01F 9/08

(54) **POLYTITANSÄUREESTER UND DEREN VERWENDUNG ZUR HERSTELLUNG VON IMPLANTIERBAREN, GGF. RESORBIERBAREN FASERN**
POLYTITANIC ACID ESTERS AND USE THEREOF TO PRODUCE IMPLANTABLE, OPTIONALLY ABSORBABLE FIBERS
ESTERS D'ACIDE POLYTITANIQUE ET UTILISATION DESDITS ESTERS D'ACIDE POLYTITANIQUE POUR LA PRODUCTION DE FIBRES IMPLANTABLES, LE CAS ÉCHÉANT RÉSORBABLES

(30) Priorität: 17.02.2011 DE 102011011544
(43) Veröffentlichungstag der Anmeldung: 25.12.2013
(73) Patentinhaber: Fraunhofer-Gesellschaft zur Förderung der angewandten Forschung e.V., 80686 München (DE)
(72) Erfinder: JAHN, Rainer, 97299 Zell (DE); ROTHENBURGER-GLAUBITT, Miranda, 97276 Margetshöchheim (DE); GLAUBITT, Walther, 97276 Margetshöchheim (DE); PROBST, Joern, 97273 Kuernach (DE)
(74) Vertreter: Olgemöller, Luitgard Maria
(86) Internationale Anmeldenummer: PCT/EP2012/052513
(87) Internationale Veröffentlichungsnummer: WO 2012/110512

(56) Entgegenhaltungen:
- US-A- 2 870 181
- K. NAKANE ET AL.: JOURNAL OF APPLIED POLYMER SCIENCE, Bd. 104, 2007, Seiten 1232-1235, XP002673527, in der Anmeldung erwähnt

## Beschreibung

Die vorliegende Erfindung betrifft Polytitansäureester sowie eine spinnfähige Masse, die sich durch Hydrolyse und Kondensation einer Titan-Koordinationsverbindung mit weniger als zwei 2-Hydroxycarbonsäure-Anionen pro Titanatom herstellen lässt und diese enthält. Insbesondere kann die Masse wasserlösliches Polypropionsäure-2-oxotitanoxidhydrat oder ein Salz davon enthalten. Daraus verspinnbare Fasern eignen sich als resorbierbares Implantatmaterial für die Medizintechnik, beispielsweise zur Verwendung als Stützkörper für die Neubildung von Knochen-, Knorpel- oder Weichgewebezellen.

Resorbierbare Implantate werden seit Jahrzehnten in der Implantologie eingesetzt. Bekannt wurden resorbierbare Biomaterialien mit dem Aufkommen von sich selbst auflösenden Nahtfäden auf der Basis von synthetisch hergestellten Polylactiden (PLA) und Polyglycoliden (PGLA) in den 70er Jahren des letzten Jahrhunderts. In einer nächsten Generation wurden aus diesem Material Implantate wie Schrauben und Platten für Gewebe- und Knochenfixierungen hergestellt. Mit zunehmender Kenntnis über Materialeigenschaften, Degradationsverhalten und Machbarkeit verschiedener klinischer Indikationen gelang es in den 90er Jahren, erste Gefäßstützen (Stents), Weichgewebe-Verstärkungsimplantate (z.B. Herniennetze) und Membransysteme, wie sie u.a. in der Kieferchirurgie verwendet werden, herzustellen. Weitere Beispiele aus jüngerer Vergangenheit sind Hydrogel-gefüllte Röhrchen als sogenannte Nervenleitschienen im Bereich der neuronalen Regeneration oder Zellträgerstrukturen für den Bereich Tissue Engineering.

Trägermaterialien für das Tissue Engineering enthalten als Grundbestandteil meistens organische, resorbierbare Polymere (PLA/PGLA). Die anfängliche Festigkeit der Struktur und somit die Formgebung wird zunächst durch den resorbierbaren Stützkörper erzielt. Nach einer gewissen Proliferationsphase von Knochen-, Knorpel- oder Weichgewebezellen nimmt deren Eigenbelastbarkeit zu und der Trägerkunststoff des Stützkörpers löst sich wiederum allmählich auf, bis nur noch reines Zellgewebe bzw. Knochenmaterial vorhanden ist. Problematisch bei den resorbierbaren Implantaten nach dem Stand der Technik ist die Tatsache, dass das Implantat aufgrund seines Materials vom Empfängerkörper als Fremdkörper erkannt wird und eine sterile Immunantwort auslöst. Dies führt zwar in der Regel nicht zu Abstoßungsreaktionen, wie sie bei allogenen (d.h. von einem menschlichen Fremdspender stammenden) oder xenogenen (von einem tierischen Spender stammenden) Organimplantaten zu beobachten sind; jedoch können die Anlagerung und das Wachstum der körpereigenen Zellen im Bereich des Implantates dadurch beeinträchtigt werden.

Resorbierbare Implantate wie Nahtmaterialien aus PLA konnten verbessert werden, indem eine sehr dünne, nicht resorbierbare Titanbeschichtung aufgebracht wurde. Die nicht resorbierbare Beschichtung bedeckt das resorbierbare Material nur teilweise. Im beschichteten Bereich werden eine Anlagerung und das Wachstum der körpereigenen Zellen unterstützt, wodurch ein schnelleres Einheilen ermöglicht wird. Auf dem beschichteten Teil des Implantats wird die Resorption verlangsamt, siehe EP 1 752 167 B1.

Am Fraunhofer-Institut für Silikatforschung wurden biologisch degradierbare bzw. resorbierbare Fasern der formalen chemischen Zusammensetzung Siₙ(OH)₂ₓO₂ₙ₋ₓ hergestellt. Die mit den Fasern erzeugten Verbundwerkstoffe, beispielsweise eine resorbierbare Wundauflage, sind in ihren mechanischen Eigenschaften denen der reinen Polymerkompaktwerkstoffe deutlich überlegen. So hält die Wundauflage ihre Stützfunktion wesentlich länger aufrecht als ein herkömmliches organisches Implantat. Adhärierung und Proliferation von Zellen werden nicht beeinträchtigt. Die Abbaurate (Löslichkeit) wird durch den Silanolgehalt bestimmt, der durch die Herstellungsparameter (Wassergehalt, Lagerung) einstellbar ist.

Fasern aus kommerziell erhältlichem Titan-bis-(ammoniumlactato)-dihydroxid in Kombination mit Polyvinylalkohol (PVA) als Spinnhilfsmittel lassen sich über ein Elektrospinnverfahren herstellen. Durch Calzinieren wurden aus diesen Fasern TiO₂-Fasern hergestellt, die sich für photokatalytische Zwecke eigneten (K.K. Nakane et al., Journal of Applied Polymer Science, Vol. 104, 1232-1235 (2007)). Das Elektrospinnverfahren wurde von T. Schiestel am Fraunhofer-Institut für Grenzflächen und Bioverfahrenstechnik (IGB) zur Herstellung sehr dünner Fasern eingesetzt. Dabei wird der Jet einer Flüssigkeit im elektrischen Feld beschleunigt und dabei immer weiter in Strahlen aufgespalten, die sich aufgrund der gleichsinnigen Ladung gegenseitig abstoßen. Setzt man dabei Lösungen von Polymeren ein, kommt es beim Spinnen zu einer Verdampfung des Lösungsmittels, und es entstehen letztendlich Polymerfasern mit Durchmessern im Sub-Mikrometer- und Nanometerbereich. Das Elektrospinnen lässt sich für die verschiedensten Materialien wie Polyvinylpyrrolidon (PVP), PEO oder PLA anwenden. Durch den Zusatz von anorganischen Precursoren zur Spinnlösung und anschließendem Sintern lassen sich auch rein anorganische Fasern herstellen.

Es sind weitere Verfahren zur Herstellung von TiO₂-Fasern bekannt. Nach der Lehre der Patente US 6,191,067 und US 6,409,961 werden alkoholische Lösungen von Titanalkoxiden (mit und ohne Komplexierung durch β-Diketone der Formel R⁴COCH₂COR⁵, worin R⁴ und R⁵ Alkyl- oder Alkoxygruppen darstellen, oder Alkylsalicylate) hydrolysiert und eingeengt. Das dabei entstehende Polymer wird mit Tetrahydrofuran aufgenommen und versponnen. Die Fasern werden mit Wasserdampf behandelt und calziniert. Eine ähnliche Offenbarung findet sich in US 4,166,147. JP 62223323 (1987) beschreibt eine alkoholische Titanoxid-Lösung, die mit Salzsäure hydrolysiert und kondensiert wird. Aus dieser Lösung können angeblich Fasern gesponnen werden. Sie werden erhitzt, um Titanoxid-Fasern zu erhalten. In EP 1 138 634 B1 wird ein ähnliches Verfahren beschrieben. Allerdings ließen sich die Angaben dieser Druckschrift nicht zuverlässig nachvollziehen. Die Erfinder der vorliegenden Anmeldung haben nämlich festgestellt, dass die wässrige Hydrolyse von Isopropoxytitanat unter den in EP 1 138 634 B1 angegebenen Bedingungen zu einer Suspension führt, die sich nicht zu einer verspinnbaren Masse einengen lässt. Dies ist nicht überraschend, da allgemein bekannt ist, dass Titanalkoxide bei Zugabe von Wasser sofort hydrolytisch kondensieren, wobei ringförmige bzw. dreidimensionale Cluster entstehen.

Textile Strukturen aus resorbierbaren Fasern werden in großem Ausmaß als medizinische Implantate eingesetzt, um Weich- und Hartgewebe zu unterstützen oder zu ersetzen. Durch geeignete Prozessführung und Auswahl des Fasermaterials ist es möglich, Oberfläche, Porosität und mechanische Anisotropie in hohem Maße zu variieren, um die einzigartigen strukturellen und mechanischen Eigenschaften von biologischem Gewebe nachzuahmen. Aktuelle Einsatzgebiete sind die Bereiche Wundversorgung (Vlies), Wundverschluss (Nahtmaterial), Herniennetze und Gefäße (Gestricke) sowie Zellträgersysteme für die Züchtung von Knochen, Haut, Knorpel, Sehnen oder Lebergewebe in vitro.

Titan ist aufgrund seiner hohen Korrosionsbeständigkeit und ausgezeichneten Bioverträglichkeit ein sehr verbreitetes Material im Bereich nicht resorbierbarer Implantate. Allein im Hüftbereich werden jährlich weltweit mehr als eine Million Endoprothesen mit Titanschaft und Hüftpfanne aus Titan eingesetzt. Seine Bioverträglichkeit verdankt Titan der nur wenige Nanometer dünnen Oxidhydratschicht an seiner Oberfläche. Humane Osteoblastenzellen strecken sich darauf regelrecht aus und verankern sich fest mit dem Werkstoff, wodurch ein direkter Kontakt zum Knochen entsteht. Fibroblasten vermehren sich unbeeinflusst und wachsen bis an die Implantatränder von mit Titanoxid passiviertem Titan, siehe Köhler S. T., Retemeyer K., Berger G., Untersuchungen zur Haftvermittlung von Bio-Vitrokeramik und Titan im tierischen Knochen, Zeitschrift für experimentelle Chirurgie 14 (1981) 139-143. Untersuchungen des Hirngewebes von Kaninchen nach Titanimplantationen zeigten keinerlei degenerativen Veränderungen, siehe Hirai H., Okumura A., Goto M., Katsuki T., Histologic study of the bone adjacent to titanium bone screws used for mandibular fracture treatment, Journal of Oral and Maxillofacial Surgery 59 (2001) 531-537 und Morra M., Cassinelli C., Cascardo G., Cahalan P., Cahalan L., Fini M., Giardino R., Surface engineering of titanium by collagen immobilization. Surface characterization and in vitro and in vivo studies, Biomaterials 24 (2003) 4639-4654.

Derivate der Milchsäure (2-Hydroxypropionsäure) sind in der Medizintechnik ebenfalls weit verbreitet. Diezbezüglich kann beispielsweise auf Lücke, A., J. Tessmar, et al., Biodegradable poly(D,L-lactic acid)-poly(ethylene glycol)-monomethyl ether diblock copolymers: structures and surface properties relevant to their use as biomaterials, Biomaterials 21(23) (2000) 2361-70 verwiesen werden. So wird PLA aufgrund seiner Biokompatibilität und Abbaubarkeit (Degradierbarkeit) im menschlichen Körper für zahlreiche medizinische Anwendungen verwendet. PLA, oft in Verbindung mit einem Copolymer, eignet sich zum Beispiel als Nahtmaterial. Es werden daraus aber auch Nägel und Schrauben, Platten oder Stents hergestellt. Abhängig von der chemischen Zusammensetzung und Porosität kann PLA einige Monate bis zu mehreren Jahren im Körper verbleiben, bis es abgebaut ist.

Titankomplexe mit Milchsäure sind bereits in US 2,870,181 beschrieben; dort wird ihre Verwendbarkeit als Gelbildner für Polyhydroxyverbindungen wie Stärke und Polyvinylalkohol diskutiert.

Aufgabe der vorliegenden Erfindung war es, ein resorbierbares Implantatmaterial zu entwickeln, das einerseits in Faserform gebracht werden kann und das andererseits einige Vorteile miteinander verbindet, die resorbierbare organische Implantatmaterialien aus Poly-(α-hydroxycarbonsäuren) wie PLA einerseits und Titanoxidhydrat-Oberflächen andererseits zeigen.

Viele Versuche, ein solches Material aufzufinden, scheiterten. So komplexierten die Erfinder ein Titan-Ausgangsmaterial wie Titantetraethylat mit einer α-Hydroxycarbonsäure, um eine hydrolysestabile und damit in wässrigen Medien einsetzbare Titanoxoverbindung zu erzeugen. Diese wurde mit einem bekannten Spinnhilfsmittel wie Polyvinylalkohol, Polyacrylsäure oder Polyethylenoxid vermischt, und es wurde der Versuch unternommen, hieraus Fasern zu formen, indem die entstandene Masse durch Düsen gepresst wurde. Auch wurde kommerziell erhältliches Titan-bis(ammoniumlactato)-dihydroxid mit Spinnhilfsmitteln verarbeitet und getestet. Jedoch waren alle Spinnversuche, unabhängig vom verwendeten Spinnhilfsmittel, nicht zufriedenstellend, weil es zu häufigen Fadenabrissen kam. Zudem ist der Zusatz derartiger Spinnhilfsmittel möglicherweise bedenklich, zumindest aber nicht optimal, weil dadurch der späteren Faser weitere Hilfsstoffe zugesetzt werden, deren Bioverträglichkeit nicht immer geklärt ist. Ein nachträgliches Entfernen des möglicherweise problematischen Zusatzes durch Extraktion unter Erhalt der Faserform gelang nicht. Nur durch Erhitzen konnten Spinnhilfsmittel entfernt werden, wodurch aber unlösliches, nicht resorbierbares TiO₂ entstand.

Die Erfinder versuchten deshalb, Fasern ohne Spinnhilfsmittel herzustellen.

Es gibt zwei bekannte Verbindungen des Titans, in denen das Titanatom mit der α-Hydroxycarbonsäure Milchsäure komplexiert ist, nämlich Titan-bis-(ammoniumlactato)-dihydroxid (siehe H. Möckel et al., J. Mater. Chem., 1999 (99), 3051-3056), das als 50%ige wässrige Lösung im Handel ist, und Ammoniumtrilactatotitanat (Inorg. Chem. 2004, Bd. 43, 4546-4548). Das erstere der beiden Moleküle hat nach Angabe der Autoren einen monomeren Aufbau mit zwei chelatisierend komplexierenden Ammoniumlactat-Anionen und zwei gebundenen Hydroxy-Gruppen. Das letztere, ebenfalls wasserlösliche, existiert gemäß Röntgenstrukturanalyse zumindest im Festzustand in Form einer mit drei Lactatmolekülen komplexierten, monomeren Verbindung, worin alle drei Lactatmoleküle zweizähnig über Sauerstoff einen 5-Ring mit dem Titanatom bilden. Es weist demnach keine potentiellen Substituenten, z.B. eine OH- oder Alkoholat-Gruppe auf, die eine Oligomerisierung erlauben würden. Diese Verbindung musste deshalb von vorneherein außer Betracht bleiben. Titan-bis-(ammoniumlactato)-dihydroxid trägt dagegen zwei Hydroxygruppen und kann daher zu Polymerketten kondensieren. Sie sind notwendig, um ein spinnfähiges Material ohne Spinnhilfsmittel zu erhalten. Durch Einengen einer wässrigen Lösung von Titan-bis-(ammoniumlactato)-dihydroxid konnte demnach erwartungsgemäß eine hochviskose Masse erzeugt werden, aus der Fäden mit einem Glasstab gezogen werden konnten. Die dünnen Fäden zogen sich jedoch sofort zu einem kurzen, dicken Strang zusammen. Deshalb eignet sich eine derartige Masse nicht zur Faserherstellung aus einer wässrigen/(alkoholischen Lösung durch Spinnen mit Hilfe von Spinndüsen. Vermutlich bilden sich während des Abdampfens und Aufkonzentrierens der Lösung keine oder nur unzureichende linearpolymere Strukturen. Die OH-Gruppen am Ti-Atom kondensieren aufgrund von zwei Ammoniumlactato-Substituenten am Ti-Zentralatom offenbar nur wenig, und die sterische Hinderung ist so groß, dass die Oligomerisierung für eine stabile Fadenbildung nicht genügt.

Überraschenderweise ist es den Erfindern dennoch gelungen, die Aufgabe der Erfindung zu lösen und eine spinnfähige Masse mit den gewünschten Eigenschaften zu erhalten, und zwar durch die Umsetzung einer in Alkoholen oder Wasser löslichen, reaktiven Titanverbindung des formal vierwertigen Titans mit einer Verbindung (II), die folgende Struktureinheit enthält:

[O=CR⁴-(CR⁵R³)ₚ-CR¹R²-O]⁻

sofern die Titanverbindung mit der Verbindung (II) in einem Molverhältnis von 1 zu 0,5 bis 1,9, vorzugsweise von 1 zu 0,7 bis 1,5 umgesetzt wird, bezogen auf eine unverbrückte, d.h. ein einziges Titanatom enthaltende (monomere) Titanverbindung, und gegebenenfalls eine anschließende Hydrolyse des gebildeten Chelatkomplexes, um die erforderlichen Kondensationsprozesse zu ermöglichen. Sofern das Ausgangsmaterial noch keine Ti-OH-Gruppen aufweist, wird hierfür während oder nach der Umsetzung mit der die genannte Struktureinheit enthaltenden Verbindung Wasser hinzugefügt. Dabei entstehen Titansäureester mit zumindest teilweise jeweils nur einer der oben definierten Struktureinheit pro Titanatom sowie am Titanatom gebundenen Hydroxygruppen. Titansäureester mit nur einer wie oben definierten Struktureinheit besitzen die theoretische Formel Ti(O=CR⁴-(CR⁵R³)ₚ-CR¹R²-O)(OH)₃. Diese können in bekannter Weise Oxo-Verbrückungen zu weiteren Titanatomen ausbilden, sind also frei für eine zumindest teilweise lineare Polymerisation. Durch diese Kondensationsreaktion bilden sich die erfindungsgemäßen polymeren Titansäureester, die, ggf. nach Abziehen eines Teils des Lösungsmittels, in dem sie hergestellt wurden, als viskose Masse vorliegen, aus der sich über eine Spinndüse stabile Fäden spinnen lassen.

In einer ersten Variante (Variante "a") ist das Strukturelement Bestandteil einer Hydroxycarbonsäure. In diesen Fällen sind die Substituenten und Indices wie folgt definiert: R¹ ist Wasserstoff, Alkyl oder Alkenyl, vorzugsweise Wasserstoff, C₁-C₆-Alkyl oder C₁-C₆-Alkenyl, besonders bevorzugt Wasserstoff oder C₁-C₄-Alkyl, R² ist Wasserstoff, Alkyl oder Alkenyl, vorzugsweise Wasserstoff, Methyl oder Ethyl, und R³ und R⁵ sind unabhängig voneinander Wasserstoff oder ein unsubstituiertes oder mit vorzugsweise OH substituiertes Alkyl oder Alkenyl, stärker bevorzugt mit jeweils 1 bis 4 Kohlenstoffatomen. R⁴ ist eine Hydroxygruppe oder ein entsprechendes Salz OM mit M = einwertiges Kation wie Li, Na, K, NH₄, oder OM_{1/2} mit M = zweiwertiges Kation wie Mg oder Ca, wobei ein solches Kation dann zwei Moleküle des Strukturelements koordinieren kann. Der Index p kann 0 oder 1 sein, wobei es für den Fall, dass p = 1 ist, günstig ist, wenn mindestens einer der Reste R³ und R⁵ nicht Wasserstoff ist. Vorzugsweise ist R⁵ in diesem Fall optional mit Hydroxy substituiertes Methyl oder Ethyl.

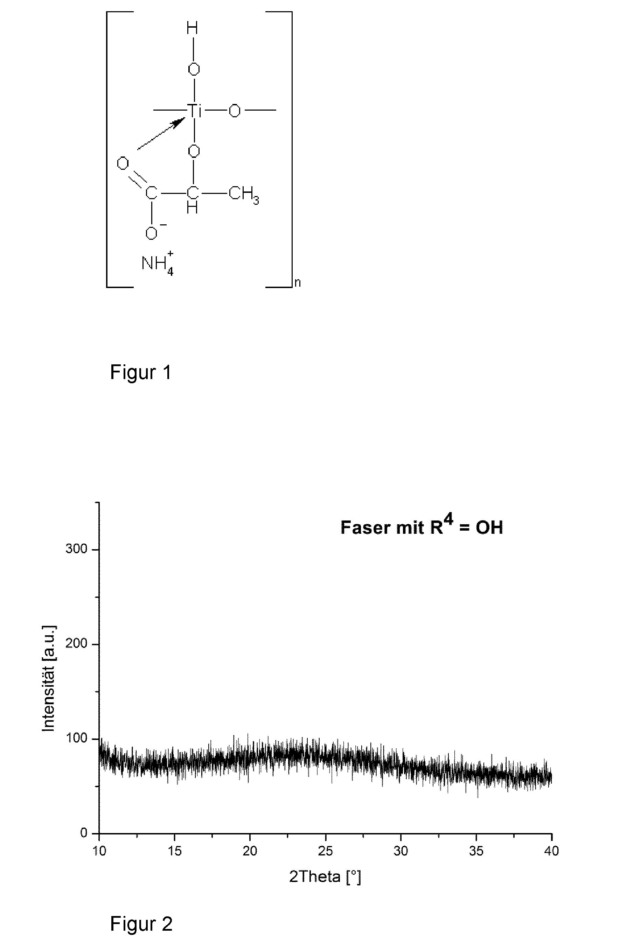

Wird eine Säure gemäß dieser ersten Variante mit der Titan-Ausgangsverbindung umgesetzt, bildet sie mit dem Titanatom wahrscheinlich einen fünf- bzw. sechsgliedrigen Chelatring, in dem sie über das Anion der Hydroxygruppe und die C=O-Gruppe der Carboxygruppe am Titanatom komplexiert ist, siehe **Figur 1****.** Diese Annahme basiert auf mehreren Gründen: Zum einen wird in der Literatur eine solche Komplexierung für die beiden Milchsäuregruppen des Moleküls Bis(ammoniumlactato)-titandihydroxid angegeben (siehe Möckel et al., a.a.O.), und zum anderen haben die Erfinder für Verbindungen dieses Typs einen sehr niedrigen pKₛ gemessen. Hierfür dürfte das Proton der Carboxygruppe verantwortlich sein, das durch Umsetzung mit einer Base ggf. erfolgreich gegen ein anderes Kation wie Natrium, Kalium oder Ammonium ausgetauscht werden kann.

In bevorzugten Ausführungsformen der ersten Variante der Erfindung ist die Verbindung mit der genannten Struktureinheit Glycolsäure (2-Hydroxyessigsäure), Milchsäure (2-Hydroxypropionsäure), 2-Hydroxybuttersäure, 2-Hydroxyisobuttersäure oder 2,2-Bis(hydroxymethyl)propionsäure. Die Umsetzung einer dieser Säuren mit Titanethylat oder dergleichen führt, ggf. nach Hydrolyse der restlichen am Titanatom vorhandenen Gruppen, zu einem Produkt, das man wahrscheinlich als Carbonsäure-2- oder 3-oxotitanat-trihydroxid bezeichnen kann, beispielsweise als Essigsäure-, Propionsäure- oder 2-Hydroxymethyl-2-methyl-propionsäure-3-oxotitanat-trihydroxid. Die beim Austausch des freien Säureprotons gegen ein Alkali- oder Ammoniumkation entstehende Verbindung kann dann als Alkali- oder Ammoniumcarboxylat-2-oxotitanattrihydroxid bezeichnet werden. Sowohl aus den Verbindungen mit komplexierter Carbonsäuregruppe als auch aus den Verbindungen mit komplexierter Kation-Carboxylatgruppe lässt sich durch Hydrolyse eine spinnfähige Masse erhalten, die wahrscheinlich das entsprechende, aus linearen Polymerketten aufgebaute Poly(carbonsäure-2-oxo-titanoxidhydrat) bzw. Poly(alkali- oder ammoniumcarboxylat-2-oxotitanoxidhydrat) enthält. Derartige Polymere werden vorliegend als Polytitansäureester bezeichnet.

In einer zweiten Variante ("Variante b") ist die Struktureinheit Bestandteil einer Keto- oder Aldocarbonsäure. In diesem Falle bedeuten R¹ und R² zusammen =O, R⁴ ist Alkyl oder Alkylen mit vorzugsweise 1 bis 6 Kohlenstoffatomen bzw. Wasserstoff, während die übrigen Reste und Indices dieselbe Bedeutung wie für die erste Variante haben. In dieser Variante können R³ und R⁵ in günstiger Weise auch beide Wasserstoff sein. Beispielhaft seien als einsetzbare Säuren Brenztraubensäure (2-Oxopropansäure) und Glyoxylsäure (Oxoessigsäure) genannt.

Die Alkyl-, die Alkenyl- und die Alkylengruppen der vorliegenden Erfindung können in allen Ausführungsformen unabhängig voneinander geradkettig, verzweigt oder cyclisch vorliegen. Sie können substituiert oder unsubstituiert sein.

Hiervon unabhängig ist der Index p in einer bevorzugten Ausführungsform, die beide Varianten betrifft, 0.

Alle vorgenannten Ausführungsformen sind, soweit sie einander nicht ausschließen, miteinander kombinierbar.

Als titanhaltige Ausgangsverbindung eignen sich beliebige Titanverbindungen, in denen Titan in formal vierwertiger Form vorliegt. Bevorzugt sind Verbindungen, in denen nicht mehr als 2 OH-Reste vorhanden sind. Günstig sind die käuflichen Titanalkylate (Titanalkoxide) wie Titanethylat Ti(OC₂H₅)₄. Titanverbindungen ohne freie Hydroxygruppen sind besonders bevorzugt.

Neben der Verbindung mit der genannten Struktureinheit können der Umsetzung weitere Monocarbonsäuren wie Essigsäure oder Propionsäure zugesetzt werden.

Das molare Verhältnis von (monomerer) Titanverbindung zur die genannte Struktureinheit enthaltenden Verbindung (II) liegt vorzugsweise im Bereich von 1 zu 0,7 bis 1,5, stärker bevorzugt im Bereich von 1 zu 0,9 bis 1:1 und ganz besonders bevorzugt im Bereich von 1:1. Dabei wurde festgestellt, dass dann, wenn weniger als 0,5 Mol, in manchen Fällen auch bereits weniger als 0,7 Mol der die Struktureinheit enthaltenden Verbindung pro Mol Titan eingesetzt wurden, kein wasserlösliches Reaktionsprodukt erhalten wurde, weshalb ein Fadenziehen nicht möglich war. Mit einem Anteil von über 1,9 Mol der die Struktureinheit enthaltenden Verbindung pro Mol Titan konnten keine Massen hergestellt werden, aus denen sich noch Fäden hätten ziehen lassen. Im Bereich zwischen 1,5 und (häufig) 1,8, zumindest bis zu etwa 1,7 Mol der die Struktureinheit enthaltenden Verbindung pro Mol Titan ließen sich zwar Fäden ziehen, diese waren jedoch nicht in allen Fällen stabil; sie ließen sich teilweise nicht trocknen und/oder schnurrten zu Tropfen zusammen. Deshalb ist es erfindungsgemäß bevorzugt, den Anteil an die Struktureinheit enthaltender Verbindung so zu wählen, dass er nicht über 1,6, vorzugsweise nicht über 1,5 Mol pro Mol (monomerer) Titanverbindung liegt.

Wird der Reaktion weiterhin eine Monocarbonsäure zugesetzt, so ist es empfehlenswert, dass auch das molare Verhältnis von (monomerer) Titanverbindung zur Summe aus der Verbindung (II) und der Monocarbonsäure im oben genannten Bereich für das Verhältnis von Titanverbindung zur Verbindung (II) liegt oder dieses nur geringfügig unterschreitet.

Ansätze aus einem äquimolaren Verhältnis von Milchsäure und Titanethylat zeigen bei Röntgenkleinwinkeluntersuchungen eine oligomere Struktur von 3 nm Größe. Derartige Kolloide kristallisieren zwar nicht beim Einengen; ein Fachmann musste jedoch befürchten, dass sie während des Lösemittelverlustes zu einem dreidimensionalen, nicht spinnfähigen Gel kondensieren würden. Dies war besonders deshalb zu erwarten, weil der in Lösung befindliche Stoff drei Kondensationszentren (Hydroxygruppen) pro Zentralatom aufweist, (dies ist ganz allgemein bei Verbindungen der Fall, die nur einen Chelat-Substituenten am Titan-Atom tragen). Es war daher überraschend, dass sich Lösungen von Titanverbindungen, die den obigen Kriterien genügen, zu stabilen, spinnfähigen Massen einengen lassen. Die Erfinder nehmen an, dass sich in der jeweiligen wasserhaltigen oder wässrigen Lösung ein Poly(carbonsäure-2-oxo-titanoxidhydrat) mit linearen Polymerketten ausbildet, die weder kristallisieren noch quervernetzen.

Die Umsetzung erfolgt in der Regel in Lösung, und zwar vorzugsweise in einem Alkohol oder einem Alkohol-Wasser-Gemisch, ggf. auch direkt in Wasser. Als Alkohole werden vorzugsweise bei Raumtemperatur, ggf. bei mindestens bis 60°C flüssige Alkylalkohole wie Methanol, Ethanol, n- oder i-Propanol oder n-, i- oder t-Butanol eingesetzt. Gegebenenfalls (und weniger bevorzugt) lassen sich aber auch andere polare Lösungsmittel, allein oder in Mischung mit den vorgenannten, einsetzen. Beispiele sind Ketone wie Aceton. Unabhängig von der Wahl des Lösungsmittels kann dieses weiterhin Säuren, insbesondere organische Säuren wie Milchsäure oder eine andere der für die Erfindung verwendbaren Säuren, oder Base wie z.B. Ammoniak enthalten.

Zumindest dann, wenn die titanhaltige Ausgangsverbindung keine freien Hydroxygruppen aufweist, ist die Anwesenheit oder die nachträgliche Zugabe von Wasser für die Hydrolyse zwingend erforderlich. Auch dann, wenn die titanhaltige Ausgangsverbindung nicht mehr als zwei freie Hydroxygruppen aufweist, ist dies empfehlenswert. Um hierbei eine Bildung von Titandioxid (Anatas) zu vermeiden, erfolgt die Hydrolyse vorzugsweise bei einer Temperatur, die zwischen 10 und 35°C liegt, insbesondere bei Raumtemperatur. Das Produkt liegt in Form einer manchmal trüben, manchmal klaren, gelartigen Lösung im gewählten Lösungsmittel(gemisch) vor. Diese Lösung lässt sich bei Bedarf auf die gewünschte Feststoffkonzentration und Visikosität einstellen, z.B. durch Aufkonzentrieren (wofür das gängige Einrotieren bei vermindertem Druck genutzt werden kann), ggf. auch durch Verdünnen. Günstig ist es, eine Verdampfung des Lösungsmittels zu betreiben, bis eine zähe Masse mit einem Feststoffgehalt von ca. 20-50 Gew.-%, vorzugsweise 30-35 Gew.-% entsteht, wobei der Feststoffgehalt hier definiert ist als der (theoretische) Titandioxidgehalt. Auch für diesen Schritt sollte man zu hohe Temperaturen vermeiden; günstig ist es, mit Badtemperaturen im Bereich von 30-70°C, stärker bevorzugt von ca. 50°C zu arbeiten.

Zur Faserherstellung kann die viskose Masse durch Düsen gepresst oder anderweitig zu Fäden gezogen werden. In Betracht kommen hierfür vor allem Düsen mit einem Durchmesser zwischen 50 µm und 1000 µm. Die Viskosität liegt im Bereich von 30 bis 250 Pa·s (20,0 °C). Die höherviskosen Ansätze können durch Erwärmen auf 40 °C auch durch geringere Düsendurchmesser (150 µm) versponnen werden. Durch die Einstellung der Spinntemperatur, der Viskosität (als günstig hat sich der Bereich von 30-50 Pa·s erwiesen), der Konzentration der Spinnmasse und des Düsendurchmessers können die Durchmesser der erhaltenen Fasern in einem weiten Bereich eingestellt werden, wobei ein Bereich von 5 bis 200 µm besonders günstig ist. Die gebildeten Fasern lassen sich ggf. nach Trocknen als Endlosfasern aufwickeln oder, z.B. nach Schneiden, anderweitig lagern, z.B. in Form eines Vlieses. Für das Antrocknen eignet sich beispielsweise eine Fallstrecke, die in günstiger Weise mehrere Dezimeter bis Meter lang ist.

Die durch das Spinnen erhaltenen Fasern sind sehr gut wasserlöslich und können in kolloidales Titanoxidhydrat, dieselbe Verbindung wie sie auf der Oberfläche von Titanimplantaten vorhanden ist, sowie die chelatisierende Verbindung bzw. deren Salz, wenn deren saures Proton gegen ein Salz-Kation ausgetauscht wurde, zerfallen. Wenn die chelatisierende Verbindung eine biologisch verträgliche oder sogar resorbierbare Verbindung wie Milchsäure bzw. ein Salz davon wie Ammoniumlactat ist, kann diese ggf. sogar in Wechselwirkung mit körpereigenen Stoffen in Abbauprodukte wie Natrium- oder Calciumlactat zerfallen. Das sind Stoffe, die von der US-Gesundheitsbehörde (Food and Drug Administration, FDA) als generell unbedenklich ("generally recognized as safe) eingestuft werden. Solche unbedenklichen Zerfallsprodukte können ggf. auch durch Zugabe von Calcium- oder Magnesiumhydroxid zu den Spinnansätzen erzeugt werden.

Die Wasserlöslichkeit der erhaltenen Fasern kann dadurch verändert werden, dass sie einem spezifischen Temperaturregime ausgesetzt werden. Werden sie stärker erhitzt, nimmt ihre Wasserlöslichkeit ab. Durch mehrstündiges Ausheizen bei Temperaturen im Bereich von 100 bis 300°C, z.B. bei 200°C für ca. 20-30 Stunden, können sie völlig wasserunlöslich gemacht werden.

Zahlreiche in vivo und in vitro durchgeführte Untersuchungen haben die Biokompatibilität von amorphem Titanoxidhydrat auf Titanimplantaten bereits bestätigt. Während Fasern aus reinen organischen Polymeren wie z.B. Polyglycosiden, D/L-Polylactiden oder anorganischem Calciumphosphat rekristallisieren und so Fremdkörper-Abwehrreaktionen hervorrufen können und/oder einem Abbau unterworfen sind, dessen Degradationsprodukte am Implantationsort eine intolerable pH-Verschiebung ins Saure verursacht, lässt sich die erfindungsgemäße Faser in einer Ausführungsform der Erfindung in pH-neutraler Form herstellen, so dass sie weder hinsichtlich ihrer Abbauprodukte negative Auswirkungen auf das Gewebemilieu noch das Auftreten von Fremdkörperreaktionen erwarten lässt. Wird stattdessen das saure Material für die Faser verwendet, kann die Säuregruppe zur Ankopplung von Wirkstoffen genutzt werden.

Nachstehend soll die Erfindung anhand von **Beispielen** näher erläutert werden.

### 1. Herstellung spinnfähiger Massen

### Beispiel 1.1

Variante a; R⁴ = OH; R¹ = H; R² = CH₃; p gleich 0; Milchsäuregehalt:1 mol; hoher Wassergehalt

230,35 g (5,00 mol) Ethanol werden bei Raumtemperatur zu 233,0 g (1,00 mol) Titanethylat gegeben. Nachdem sich eine klare Lösung gebildet hat, werden 105,98 g (1,00 mol) einer 85%igen Milchsäurelösung bei Raumtemperatur zugegeben. Das Reaktionsgemisch reagiert leicht exotherm. Nach Abkühlen auf Raumtemperatur werden 333,33 g (18,5 mol) Wasser auf einmal zugegeben, was zu einer Trübung des Gels und einer Bildung eines weißen Niederschlages führt. Das Reaktionsgemisch wird für 24 h bei Raumtemperatur gerührt; dabei löst sich der Niederschlag wieder auf. Die Mischung wird im Wasserbad (50 °C) bei einem Druck von bis zu 40 mbar einrotiert bis eine zähe Masse entsteht.

### Beispiel 1.2

Beispiel 1.1 wurde mit der Maßgabe wiederholt, dass anstelle von Milchsäure der entsprechende molare Anteil an Glycolsäure eingesetzt wurde.

### Beispiel 1.3

Variante a; R⁴ = OH; R¹ = H; R² = CH₃; p gleich 0; Milchsäuregehalt: 1 mol; niedriger Wassergehalt 230,35 g (5,00 mol) Ethanol werden bei Raumtemperatur zu 233,0 g (1,00 mol) Titanethylat gegeben. Nachdem sich eine klare Lösung gebildet hat, werden 105,98 g (1,00 mol) einer 85%-igen Milchsäurelösung bei Raumtemperatur zugegeben. Das Reaktionsgemisch reagiert leicht exotherm. Nach Abkühlen auf Raumtemperatur werden 9,01 g (0,5 mol) Wasser auf einmal zugegeben, was zu einer Trübung des Gels und einer Bildung eines weißen Niederschlages führt. Das Reaktionsgemisch wird für 24 h bei Raumtemperatur gerührt; dabei löst sich der Niederschlag wieder auf. Die Mischung wird im Wasserbad (50 °C) bei einem Druck von bis zu 40 mbar einrotiert, bis eine zähe Masse entsteht.

### Beispiel 1.4

Variante a; R⁴ = OH; R¹ = H; R² = CH₃; p gleich 0; Milchsäuregehalt: 0,75 mol; niedriger Wassergehalt

230,35 g (5,00 mol) Ethanol werden bei Raumtemperatur zu 233,0 g (1,00 mol) Titanethylat gegeben. Nachdem sich eine klare Lösung gebildet hat, werden 79,48 g (0,75 mol) einer 85%-igen Milchsäurelösung bei Raumtemperatur zugegeben. Das Reaktionsgemisch reagiert leicht exotherm. Nach Abkühlen auf Raumtemperatur werden 9,01 g (0,5 mol) Wasser auf einmal zugegeben, was zu einer Trübung des Gels und einer Bildung eines weißen Niederschlages führt. Das Reaktionsgemisch wird für 24 h bei Raumtemperatur gerührt, dabei löst sich der Niederschlag wieder auf. Die Mischung wird im Wasserbad (50 °C) bei einem Druck von bis zu 40 mbar einrotiert, bis eine zähe Masse entsteht.

### Beispiel 1.5

Variante a; R⁴ = OH; R¹ = H; R² = CH₃; p gleich 0; Milchsäuregehalt: 0,5 mol; niedriger Wassergehalt

230,35 g (5,00 mol) Ethanol werden bei Raumtemperatur zu 233,0 g (1,00 mol) Titanethylat gegeben. Nachdem sich eine klare Lösung gebildet hat, werden 52,99 g (0,50 mol) einer 85%-igen Milchsäurelösung bei Raumtemperatur zugegeben. Das Reaktionsgemisch reagiert leicht exotherm. Nach Abkühlen auf Raumtemperatur werden 9,01 g (0,5 mol) Wasser auf einmal zugegeben, was zu einer Trübung des Gels und einer Bildung eines weißen Niederschlages führt. Das Reaktionsgemisch wird für 24 h bei Raumtemperatur gerührt, dabei löst sich der Niederschlag wieder auf. Die Mischung wird im Wasserbad (50 °C) bei einem Druck von bis zu 40 mbar einrotiert, bis eine zähe Masse entsteht.

### Beispiel 1.6

Variante a; R⁴ = OM mit M = NH₄⁺; R¹ = H; R² = CH₃; p gleich 0; Milchsäuregehalt: 1 mol; hoher Wassergehalt 230,35 g (5,00 mol) Ethanol werden bei Raumtemperatur zu 233,0 g (1,00 mol) Titanethylat gegeben. Nachdem sich eine klare Lösung gebildet hat, werden 105,98 g (1,00 mol) einer 85%-igen Milchsäurelösung bei Raumtemperatur zugegeben. Das Reaktionsgemisch reagiert leicht exotherm. Nach Abkühlen auf Raumtemperatur werden 333,33 g (18,5 mol) Wasser auf einmal zugegeben, was zu einer Trübung des Gels und einer Bildung eines weißen Niederschlages führt. Das Reaktionsgemisch wird für 24 h bei Raumtemperatur gerührt, dabei löst sich der Niederschlag wieder auf. Das Sol wird mit 47,68 g (0,70 mol) Ammoniakwasser (25%) neutralisiert. Die durch die exotherme Reaktion erwärmte Reaktionsmischung wird bis zum Abkühlen auf Raumtemperatur gerührt. Daraufhin wird die Mischung im Wasserbad (50 °C) bei einem Druck von bis zu 40 mbar einrotiert, bis eine zähe Masse entsteht.

Die vorgenannten Mengenverhältnisse wurden im Bereich von 0,5 bis 1,8 Mol Milchsäure pro Mol Titanverbindung variiert. Aus Massen, denen ein Mengenverhältnis von zwischen 0,7 und 1,5 Mol Milchsäure pro Mol Titanverbindung zugrunde lag, ließen sich problemlos Fäden spinnen.

### Beispiel 1.7

Variante a; R⁴ = OM mit M = NH₄⁺; R¹ = H; R² = H; p gleich 0; Milchsäuregehalt: 1 mol; hoher Wassergehalt
Beispiel 1.6 wurde mit der Maßgabe wiederholt, dass anstelle von Milchsäure Glycolsäure eingesetzt wurde.

### Beispiel 1.8

Variante a; R⁴ = OM mit M = NH₄⁺; R¹ = H; R² = (CH₃; H); p gleich 0; Milchsäuregehalt: 1 mol; hoher Wassergehalt
Die Herstellung erfolgte analog zu den Beispielen 1.6 und 1.7, wobei jedoch eine Mischung von Milchsäure und Glycolsäure im Verhältnis von 1:0,5 eingesetzt wurde.

### Beispiel 1.9

Variante (a); R⁴ = OM mit M = NH₄⁺; R¹ = H; R² = CH₃; p gleich 0;_unter Zusatz einer organischen Säure (Propionsäure)

1 mol (233 g) Titanethylat wird mit 230 g Ethanol gemischt, 0,75 mol (79,48 g) Milchsäure (85%) werden unter Rühren in ca. 10 Portionen zugegeben. Die Mischung wird anschließend 2 h lang gerührt. Nach Zugabe von 0,25 mol (18,52 g) Propionsäure wird eine weitere Stunde gerührt. 18,5 mol (333 g) Wasser werden unter starkem Rühren auf einmal zugeschüttet, worauf die Lösung trüb wird und teilweise eine Gelbildung beobachtet wird. Die Mischung wird im Wasserbad (50°C) bei einem Druck von bis zu 40 mbar einrotiert. Dabei klart sie auf. 0,19 mol (12,94 g) 33-%iger Ammoniak werden zugegeben, und das Einrotieren wird fortgesetzt, bis eine zähe Masse mit einer Viskosität von 40 Pa·s und mit einem Oxidgehalt von ca. 36 % Titandioxid entsteht.

### Beispiel 1.10

Variante a, R⁴ = OH; R¹ = CH₃; R² = CH₃; p gleich 0; 1,5 mol 2-Hydroxyisobuttersäure; hoher Wassergehalt

Zu einer Lösung von 11,65 g (0,05 mol) Titanethylat in 11,52 g Ethanol wird bei Raumtemperatur 7,81 g (0,075 mol) 2-Hydroxyisobuttersäure gegeben. Zu der klaren Lösung wird 16,65 g (0,92 mol) Wasser und 3,41 g (0,05 mol) 25-%-igen Ammoniak hinzugefügt. Nach Einengen am Rotationsverdampfer bei 50 °C und 50 mbar erhält man eine viskose Masse, aus der Fäden gezogen werden können.

### Beispiel 1.11

Variante a; R⁴ = OH; R¹ = H; R² = CH₃; R³ = H; R⁵ = CH₂OH; p = 1; 1,5 mol 2,2-Bis(hydroxymethyl)-propionsäure

Zu einer Lösung von 11,65 g g (0,05 mol) Titanethylat in 11,52 g Ethanol wird bei Raumtemperatur 10,06 g (0,075 mol) 2,2-bis(Hydroxymethyl)-Propionsäure gegeben. Es entsteht ein weißer Niederschlag, der sich nach Zugabe von 16,65 g (0,92 mol) Wasser und 3,41 g (0,05 mol) 25-%-igen Ammoniak auflöst. Durch Erwärmen auf 50 °C klart die Lösung vollständig auf. Nach Einengen am Rotationsverdampfer bei 50 °C und 50 mbar erhält man eine viskose Masse, aus der Fäden gezogen werden können

### Beispiel 1.12

Variante b; R⁴ = CH₃, R₁ und R₂ zusammen gleich =O; p gleich 0; 1,5 Mol Brenztraubensäure; hoher Wassergehalt

Zu einer Lösung von 11,65 g (0,05 mol) Titanethylat in 11,52 g Ethanol wird bei Raumtemperatur 6,65 g (0,075 mol) Brenztraubensäure gegeben. Es entsteht eine braune, klare Lösung, nach Zugabe von 16,65 g (0,92 mol) Wasser und 3,41 g (0,05 mol) 25-%-igen Ammoniak entsteht ein Gel, das sich durch Erwärmen auf 50 °C auflöst. Nach Einengen am Rotationsverdampfer bei 50 °C und 50 mbar erhält man eine viskose Masse, aus der bei 50 °C kurze Fäden gezogen werden können.

### Beispiel. 1.13

Variante b; R⁴ gleich H; R₁ und R₂ zusammen gleich =O und p gleich 0; 1 mol Glyoxylsäure (Oxoessigsäure); hoher Wassergehalt

Zu einer Lösung von 11,65 g (0,05 mol) Titanethylat in 11,52 g Ethanol wird bei Raumtemperatur 4,60 g (0,05 mol) Glyoxylsäure gegeben. Es entsteht eine gelbliche, klare Lösung, nach Zugabe von 16,65 g (0,92 mol) Wasser und 3,41 g (0,05 mol) 25-%-igen Ammoniak entsteht ein weißer Niederschlag. Nach Einengen am Rotationsverdampfer bei 50 °C und 75 mbar klart der Ansatz auf und man erhält eine gelige Masse, aus der kurze Fäden gezogen werden können.

### Vergleichsbeispiel:

### Hydrolyse von Titanisopropylat ohne komplexierendes Agens

Zu 14,80 g 2-Propanol wurden 60,10 g Ti(OiPr)₄ gegeben und bei Raumtemperatur gerührt. Nach der Zugabe von 7,01 g Wasser fällt sofort ein weißer Niederschlag aus. Die Suspension wurde für 1 h bei Raumtemperatur gerührt und anschließend bis zur Trockene eingeengt. Der erhaltene Feststoff wurde in ca. 300 mL THF aufgenommen und die dabei entstandene gelbliche Suspension für 2 h bei Raumtemperatur gerührt. Das Einengen der Suspension am Rotationsverdampfer führte zu einem direkten Übergang einer Suspension zu einem gelblichen Feststoff.

### 2. Faserherstellung

Die viskose Masse der Beispiele 1.1 bis 1.13 wurde jeweils bei 25°C und einem Druck von 20 bar durch eine oder mehrere 200 µm-Düsen gepresst. Nach 2,5 m Fallhöhe wurden die Fasern auf einen rotierenden Zylinder aufgewickelt oder mit einem Changiertisch (eine in 2 Dimensionen bewegliche horizontale Fläche) als Vlies abgelegt werden. Der Faserdurchmesser liegt in der Regel zwischen 20 und 100 µm.

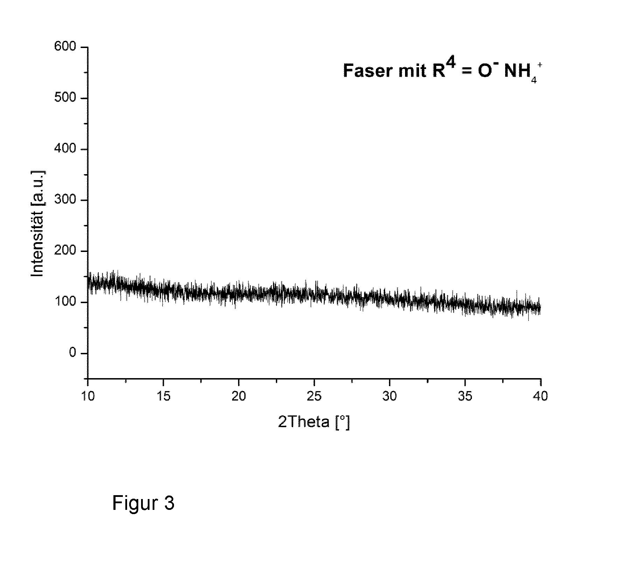

Ein Pulverröntgendiffraktogramm von auf diese Weise erhaltenen Fasern gemäß Beispiel 1.1 ist in Figur 2 und ein solches gemäß Beispiel 1.7 ist in Figur 3 gezeigt. Beide Pulver-Röntgendiffraktogramme zeigen nicht den für Anatas typischen Hauptreflex bei 25,3° 2Theta.

### 3. Weiterverarbeitung

Fasern aus den Massen des Beispiels 1.1 wurde bei 200°C an Luft in einem Trockenschrank gehalten. Nach einer Heizperiode von 2h benötigten 50 mg der Fasern mehrere Stunden, um sich in 10 g Wasser bei Raumtemperatur auflösen zu lassen. Nach 24h bei 200 °C waren sie wasserunlöslich.

## Patentansprüche

1. Polytitansäureester mit linearen Vernetzungsstrukturen, erhältlich durch Umsetzung einer in einem Alkohol, in Wasser oder in einer Mischung aus Wasser und Alkohol löslichen Titanverbindung der Zusammensetzung (I)
Ti(R)ₘXₙ (I)
worin die Reste R unabhängig voneinander Alkoxy, Carboxy oder OH bedeuten, X Halogen bedeutet, m = 0 bis 4 ist, n = 0 bis 4 ist und m + n = 4 ist,
mit einer Verbindung (II), die die folgende Struktureinheit enthält:
[O=CR⁴-(CR⁵R³)ₚ-CR¹R²-O]⁻
worin p 0 oder 1 ist und worin
(a) R¹ und R² jeweils unabhängig voneinander ausgewählt sind unter Wasserstoff, Alkyl und Alkenyl,
R³ und R⁵ unabhängig voneinander ausgewählt sind unter Wasserstoff und einem unsubstituierten oder substituierten Alkyl oder Alkenyl,
R⁴ OH oder O(M^{x+})_{1/x} mit M gleich Metall oder Ammonium und x 1 oder 2 ist
oder
(b) R¹ und R² zusammen =O bedeuten und R³, R⁵ und R⁴ jeweils unabhängig voneinander ausgewählt sind unter Wasserstoff, Alkyl und Alkenyl,
wobei die Titanverbindung (I) mit der Verbindung (II) in einem Molverhältnis von 1 zu 0,5 bis 1,9 umgesetzt wird, bezogen auf eine unverbrückte, d.h. ein einziges Titanatom enthaltende (monomere) Titanverbindung, mit der Maßgabe, dass dann, wenn die Verbindung (I) nicht mehr als zwei Hydroxygruppen aufweist und wenn die Umsetzung in einem wasserfreien Medium erfolgt, das durch die Umsetzung gebildete Produkt anschließend mit Wasser in Kontakt gebracht wird, derart, dass in allen Fällen eine hydrolytische Kondensationsreaktion der entstandenen Titanverbindung(en) erfolgt.

2. Polytitansäureester nach Anspruch 1, worin R in der Verbindung (I) Alkoxy bedeutet und/oder X Chlor bedeutet.

3. Polytitansäureester nach Anspruch 2, worin R in der Verbindung (I) Ethoxy bedeutet und/oder m = 4 ist.

4. Polytitansäureester nach einem der voranstehenden Ansprüche, worin zumindest einer der Reste R³ und R⁵ ein mit einer Hydroxygruppe substituiertes Alkyl ist.

5. Polytitansäureester nach einem der voranstehenden Ansprüche, worin die Verbindung (II) ausgewählt ist unter Hydroxycarbonsäuren.

6. Polytitansäureester nach Anspruch 5, worin der Masse während oder nach der hydrolytischen Kondensationsreaktion eine Ammoniumverbindung, Ammoniak oder ein Alkali- oder Erdalkalisalz zugesetzt wird.

7. Polytitansäureester nach einem der Ansprüche 1 bis 4, worin die Verbindung (II) ausgewählt ist unter Salzen von Hydroxycarbonsäuren.

8. Polytitansäureester nach einem der Ansprüche 1 bis 3, worin die Verbindung (II) ausgewählt ist unter Keto- und Aldocarbonsäuren.

9. Polytitansäureester nach Anspruch 1, worin die Verbindung der Formel (II) ausgewählt ist unter Glycolsäure, Milchsäure, 2-Hydroxybuttersäure, 2-Hydroxyisobuttersäure, 2,2-Bis(hydroxymethyl)propionsäure, 2-Oxopropansäure, Glyoxylsäure und Mischungen dieser Säuren.

10. Polytitansäureester nach einem der voranstehenden Ansprüche, erhältlich unter zusätzlicher Beigabe von Essigsäure und/oder Propionsäure.

11. Spinnbare Masse, umfassend einen Polytitansäureester nach einem der voranstehenden Ansprüche in einem Lösungsmittel, ausgewählt unter Wasser, Alkoholen und Ketonen sowie Mischungen der vorgenannten Lösungsmittel.

12. Spinnbare Masse nach Anspruch 11, worin das Lösungsmittel ausgewählt ist unter Wasser, Methanol, Ethanol, Propanol, Butanol sowie Mischungen der genannten Lösungsmittel.

13. Verfahren zum Herstellen von titanhaltigen Fasern, **dadurch gekennzeichnet, dass** eine viskose Masse bereitgestellt wird, die einen Polytitansäureester wie in einem der Ansprüche 1 bis 10 definiert mit einem Feststoffanteil von 20-50 Gew.-%, vorzugsweise 30-35 Gew.-% Feststoffgehalt, bezogen auf den theoretischen Titandioxidgehalt, in einem Lösungsmittel enthält, diese Masse durch Spinndüsen gepresst wird und die dabei entstandenen Fäden einer Trocknung unterworfen werden.

14. Verfahren zum Herstellen von in Wasser schlecht oder nicht löslichen, titanhaltigen Fasern, **dadurch gekennzeichnet, dass** eine viskose Masse bereitgestellt wird, die einen Polytitansäureester wie in einem der Ansprüche 1 bis 10 definiert mit einem Feststoffanteil von 20-50 Gew.-%, vorzugsweise 30-35 Gew.-% Feststoffgehalt, bezogen auf den theoretischen Titandioxidgehalt, in einem Lösungsmittel enthält, diese Masse durch Spinndüsen gepresst wird und die dabei entstandenen Fäden anschließend mindestens 5 Stunden bei mindestens 100°C, vorzugsweise bei mindestens 150°C erhitzt werden.

15. Verfahren nach Anspruch 13 oder 14, **dadurch gekennzeichnet, dass** die viskose Masse ausschließlich unter Verwendung der Titanverbindung der Zusammensetzung (I) und der genannten Verbindung (II) sowie gegebenenfalls einer Monocarbonsäure hergestellt wurde und im Übrigen nur ein oder mehrere geeignete Lösungsmittel enthält.

## Claims

1. Polytitanic acid ester having linear cross-linking structures, obtainable by reacting a titanium compound that is soluble in an alcohol, in water or in a mixture of water and alcohol and that has the composition (I)
Ti(R)ₘXₙ (I),
wherein the groups R independently of each other mean alkoxy, carboxy or OH, X means halogen, m = 0 to 4, n = 0 to 4 and m + n = 4,
with a compound (II) that contains the following structural unit:
[O=CR⁴-(CR⁵R³)ₚ-CR¹R²-O]⁻,
wherein p is 0 or 1 and wherein
(a) R¹ and R² are independently of each other selected from among hydrogen, alkyl and alkenyl,
R³ and R⁵ are independently of each other selected from among hydrogen and an unsubstituted or substituted alkyl or alkenyl,
R⁴ is OH or O(M^{x+})_{1/x}, wherein M is a metal or ammonium and x is 1 or 2,
or
(b) R¹ and R² together mean =O and R³, R⁵ and R⁴ are independently of each other selected from among hydrogen, alkyl and alkenyl,
wherein the titanium compound (I) is reacted with compound (II) at a molar ratio of from 1 to 0.5 to 1.9, relative to an unbridged titanium compound, i.e., a (monomeric) titanium compound containing a single titanium atom, provided that, if compound (I) has no more than 2 hydroxy groups and if the reaction occurs in a water-free medium, the product formed by the reaction is then brought in contact with water in such a way that a hydrolytic condensation reaction of the produced titanium compound(s) occurs in all cases.

2. Polytitanic acid ester according to claim 1, wherein R in the compound (I) means alkoxy and/or X means chlorine.

3. Polytitanic acid ester according to claim 2, wherein R in the compound (I) means ethoxy and/or m = 4.

4. Polytitanic acid ester according to any one of the preceding claims, wherein at least one of the groups R³ and R⁵ is an alkyl substituted with a hydroxyl group.

5. Polytitanic acid ester according to any of the preceding claims, wherein the compound (II) is selected from among hydroxy carboxylic acids.

6. Polytitanic acid ester according to claim 5, wherein an ammonium compound, ammonia or an alkali or alkali earth salt is added to the mass during or after the hydrolytic condensation reaction.

7. Polytitanic acid ester according to any of claims 1 to 4, wherein the compound (II) is selected from among salts of hydroxy carboxylic acids.

8. Polytitanic acid ester according to any of claims 1 to 3, wherein the compound (II) is selected from among keto- and aldo-carboxylic acids.

9. Polytitanic acid ester according to claim 1, wherein the compound with the formula (II) is selected from among glycolic acid, lactic acid, 2-hydroxybutyric acid, 2-hydroxyisobutyric acid, 2,2-bis(hydroxymethyl)propionic acid, 2-oxopropanoic acid, glyoxylic acid and mixtures of these acids.

10. Polytitanic acid ester according to any of the preceding claims, obtainable with the further addition of acetic acid and/or propionic acid.

11. Spinnable mass, comprising a polytitanic acid ester according to any of the preceding claims in a solvent, selected from among water, alcohols and ketones as well as mixtures of the aforementioned solvents.

12. Spinnable mass according to claim 11, wherein the solvent is selected from among water, methanol, ethanol, propanol, butanol as well as mixtures of the mentioned solvents.

13. Method for the manufacture of fibers containing titanium, **characterized in that** a viscous mass is provided that comprises a polytitanic acid ester as defined in any of claims 1 to 10, having a solid matter content of 20-50 percent by weight, preferably a solid matter content of 30-35 percent by weight, with respect to the theoretical titanium dioxide content, in a solvent, said mass is extruded through spinning nozzles and the threads created thereby are subject to drying.

14. Method for the manufacture of fibers containing titanium which are poorly soluble or insoluble in water, **characterized in that** a viscous mass is provided that comprises a polytitanic acid ester as defined in any of claims 1 to 10, having a solid matter content of 20-50 percent by weight, preferably with a solid matter content of 30-35 percent by weight, with respect to the theoretical titanium dioxide content, in a solvent, said mass is extruded through spinning nozzles and the threads created thereby are subsequently heated for at least 5 hours at a temperature of at least 100°C, preferably at least 150°C.

15. Method according to claim 13 or 14, **characterized in that** the viscous mass was manufactured exclusively with the use of the titanium compound of compound (I) and the mentioned compound (II) as well as optionally with a monocarboxylic acid and otherwise only contains one or a plurality of suitable solvents.

## Revendications

1. Esters de l'acide polytitanique avec des structures de réticulation linéaires, pouvant être obtenus en faisant réagir un composé du titane de composition (I)
Ti(R)ₘXₙ (I)
soluble dans un alcool, dans de l'eau ou dans un mélange d'eau et d'alcool, les radicaux R, indépendamment les uns des autres, désignant un groupe alcoxy, carboxyle ou OH, X désignant un halogène, m = 0 à 4, n = 0 à 4 et m + n = 4, avec un composé (II) renfermant le motif structural suivant :
[O=CR⁴-(CR⁵R³)ₚ-CR¹R²-O]⁻
p étant 0 ou 1 et dans lequel
(a) R¹ et R² sont respectivement choisis indépendamment l'un de l'autre parmi l'hydrogène, un groupe alkyle et alcényle,
R³ et R⁵ sont choisis indépendamment l'un de l'autre parmi l'hydrogène et un groupe alkyle ou alcényle substitué ou non substitué,
R⁴ est OH ou O (M^{x+}) _{1/x}, M étant un métal ou l'ammonium et x étant 1 ou 2 ou
(b) R¹ et R² désignent tous deux =O et R³, R⁵ et R⁴ sont respectivement choisis indépendamment les uns des autres parmi l'hydrogène, un groupe alkyle et alcényle,
le composé du titane (I) avec le composé (II) réagissant selon un rapport molaire de 1-0,5 à 1,9, rapporté à un composé du titane non ponté, c'est-à-dire un composé du titane (monomère) contenant un seul atome de titane, à condition que, si le composé (I) ne possède pas plus de 2 groupes hydroxyle et si la réaction se déroule dans un milieu non aqueux, le produit formé par la réaction est alors amené en contact avec de l'eau de manière à ce qu'une réaction de condensation hydrolytique du ou des composés du titane obtenus se produise dans tous les cas.

2. Esters de l'acide polytitanique selon la revendication 1, dans lesquels R dans le composé (I) désigne un groupe alcoxy et/ou X désigne le chlore.

3. Esters de l'acide polytitanique selon la revendication 2, dans lesquels R dans le composé (I) désigne un groupe éthoxy et/ou m = 4.

4. Esters de l'acide polytitanique selon l'une quelconque des revendications précédentes, dans lesquels au moins l'un des radicaux R³ et R⁵ est un groupe alkyle substitué par un groupe hydroxyle.

5. Esters de l'acide polytitanique selon l'une quelconque des revendications précédentes, dans lesquels le composé (II) est choisi parmi les acides hydroxycarboxyliques.

6. Esters de l'acide polytitanique selon la revendication 5, dans lesquels on ajoute un composé ammonium, de l'ammoniac ou un sel alcalin ou alcalino-terreux à la masse pendant ou après la réaction de condensation hydrolytique.

7. Esters de l'acide polytitanique selon l'une quelconque des revendications 1 à 4, dans lesquels le composé (II) est choisi parmi les sels d'acides hydroxycarboxyliques.

8. Esters de l'acide polytitanique selon l'une quelconque des revendications 1 à 3, dans lesquels le composé (II) est choisi parmi les acides cétocarboxyliques et aldocarboxyliques.

9. Esters de l'acide polytitanique selon la revendication 1, dans lesquels le composé de formule (II) est choisi parmi l'acide glycolique, l'acide lactique, l'acide 2-hydroxybutyrique, l'acide 2-hydroxy-isobutyrique, l'acide 2,2-bis(hydroxyméthyl)propionique, l'acide 2-oxopropanoïque, l'acide glyoxylique et des mélanges de ces acides.

10. Esters de l'acide polytitanique selon l'une quelconque des revendications précédentes, pouvant être obtenus en ajoutant en outre de l'acide acétique et/ou de l'acide propionique.

11. Masse filable comprenant un ester d'acide polytitanique selon l'une quelconque des revendications précédentes, dans un solvant choisi parmi l'eau, les alcools et les cétones ainsi que des mélanges des solvants susmentionnés.

12. Masse filable selon la revendication 11, dans laquelle le solvant est choisi parmi l'eau, le méthanol, l'éthanol, le propanol, le butanol ainsi que des mélanges des solvants mentionnés.

13. Procédé pour la fabrication de fibres contenant du titane, **caractérisé en ce qu'**une masse visqueuse est fournie, laquelle contient, dans un solvant, un ester d'acide polytitanique selon l'une quelconque des revendications 1 à 10, ayant une teneur en matières solides est de 20 à 50 % en poids, de préférence une teneur en matières solides de 30 à 35 % en poids par rapport à la teneur théorique en dioxyde de titane, ladite masse étant extrudée à travers des filières de filage et les fils ainsi créés étant soumis à un séchage.

14. Procédé pour la fabrication de fibres contenant du titane qui sont faiblement solubles ou insolubles dans l'eau, **caractérisé en ce qu'**une masse visqueuse est fournie, laquelle contient, dans un solvant, un ester d'acide polytitanique selon l'une quelconque des revendications 1 à 10, ayant une teneur en matières solides est de 20 à 50 % en poids, de préférence une teneur en matières solides de 30 à 35 % en poids par rapport à la teneur théorique en dioxyde de titane, ladite masse étant extrudée à travers des filières de filage et les fils ainsi créés étant ensuite chauffés pendant au moins 5 heures à une température d'au moins 100 °C, de préférence d'au moins 150 °C.

15. Procédé selon la revendication 13 ou 14, **caractérisé en ce que** la masse visqueuse a été produite exclusivement en utilisant le composé du titane du composé de composition (I) et le composé (II) mentionné ainsi qu'un acide monocarboxylique le cas échéant, et ne contient par ailleurs qu'un ou plusieurs solvants appropriés.
